# EUROPEAN PATENT APPLICATION

(11) **EP 2 248 477 A1**
(43) Date of publication of application: **10.11.2010**
(21) Application number: 10250878.5
(22) Date of filing: 05.05.2010
(51) Int. Cl.: A61B 17/34

(54) **Surgical portal device**

(30) Priority: 06.05.2009 US 175917 P; 25.03.2010 US 731166
(71) Applicant: Tyco Healthcare Group LP, North Haven, CT 06473 (US)
(72) Inventor: Bettuchi, Michael J., Middletown, CT 06457 (US); Taylor, Eric, East Hampton, CT 06424 (US)
(74) Representative: Soames, Candida Jane

(57) **Abstract**

A surgical portal device comprising a body portion and at least one securing member is disclosed. The body portion defines a longitudinal axis and includes a proximal end, a distal end, and a lumen configured to allow a surgical instrument to pass therethrough. The at least one securing member is disposed in mechanical cooperation with the body portion and is radially expandable with respect to the body portion between an initial position and an expanded position. The at least one securing member includes a hydrophilic material and is configured to radially expand in response to contact with moisture.

## Description

### CROSS REFERENCE TO RELATED APPLICATION

The present application claims the benefit of and priority to U.S. Provisional Application Serial No. 61/175,917 filed on May 6, 2009, the entire contents of which are incorporated herein by reference.

### BACKGROUND

### Technical Field

The present disclosure relates to surgical ports. More particularly, the present disclosure relates to surgical access ports having port fixation components to secure the surgical access port relative to tissue of a patient.

### Background of Related Art

Surgical ports, such as introducers, trocars, and cannulas, permit the introduction of a variety of surgical instruments into a body cavity or opening within a patient. In procedures, such as endoscopic, laparoscopic or arthroscopic surgeries, a passage is created through tissue to access an underlying surgical site in the body. A port or cannula is positioned within the passage. Surgical instruments are introduced within the cannula to perform a surgical procedure.

It may be advantageous to provide a portal device that can be placed within an incision or body opening of a patient and removably fixed therein.

### SUMMARY

The present disclosure relates to a surgical portal device comprising a body portion and at least one securing member. The body portion defines a longitudinal axis and includes a proximal end, a distal end, and a lumen configured to allow a surgical instrument to pass therethrough. The at least one securing member is disposed in mechanical cooperation with the body portion and is radially expandable with respect to the body portion between an initial position and an expanded position. The at least one securing member includes a hydrophilic material and is configured to radially expand in response to contact with moisture.

The present disclosure also relates to a surgical method. The surgical method comprises the step of providing a surgical portal device including a body portion, a lumen extending through the body portion, and at least one securing member disposed in mechanical cooperation with the body portion. The at least one securing member includes a hydrophilic material. The surgical method also comprises the steps of positioning the surgical portal device at least partially within tissue such that the hydrophilic material contacts moisture, radially expanding the at least one securing member from an initial position to an expanded position, such that the at least one securing member expands towards surrounding tissue, introducing a surgical instrument through the lumen of the body portion, and performing a surgical task with the surgical instrument.

The present disclosure also relates to a method of securing a portion of a surgical portal device adjacent tissue. The method comprises the step of providing a surgical portal device including a body defining a longitudinal axis and a lumen extending through the body. The method also comprises the steps of applying a hydrophilic material around at least a portion of the body portion, and positioning the surgical portal device at least partially within tissue such that the hydrophilic material contacts moisture.

The invention may be described by reference to the following numbered paragraphs:-

A surgical method, comprising the steps of providing a surgical portal device, including: a body portion defining a longitudinal axis; a lumen extending through the body; and at least one securing member disposed in mechanical cooperation with the body portion, the at least one securing member including a hydrophilic material; positioning the surgical portal device at least partially within tissue such that the hydrophilic material contacts moisture; radially expanding the at least one securing member from an initial position to an expanded position, such that the at least one securing member expands towards surrounding tissue; introducing a surgical instrument through the lumen of the body portion; and performing a surgical task with the surgical instrument.

The method of paragraph [0009], further comprising the step of returning the at least one securing member to its initial position.

The method of paragraph [0009], further comprising the step of removing the surgical portal device from the tissue.

The method of paragraph [0009], wherein the radially expanding step occurs in response to the at least one securing member contacting moisture.

The method of paragraph [0009], wherein the securing member comprises at least one of a medicinal agent, a pain reliever, an anti-inflammatory, and an antibiotic.

### BRIEF DESCRIPTION OF THE DRAWINGS

Embodiments of the presently disclosed portal device are described herein with reference to the accompanying drawings, wherein:

FIG. 1 is a perspective view of a surgical portal device according to an embodiment of the present disclosure illustrating a securing member in an initial position;

FIG. 2 is a longitudinal cross-sectional view of a portion the surgical portal device of FIG. 1 within tissue;

FIG. 3 is a longitudinal cross-sectional view of the surgical portal device of FIGS. 1 and 2, illustrating a portion of the securing member in an expanded position and within tissue;

FIG. 4 is a perspective view of a surgical portal device according to another embodiment of the present disclosure, illustrating a securing member in an initial position;

FIG. 5 is a perspective view of the surgical portal device of FIG. 4, illustrating the securing member in an expanded position;

FIG. 6 is a flow chart illustrating a surgical method incorporating a surgical portal device of the present disclosure; and

FIG. 7 is another flow chart illustrating a surgical method incorporating a surgical portal device of the present disclosure.

Other features of the present disclosure will become apparent from the following detailed description, taken in conjunction with the accompanying drawings, which illustrate, by way of example, various principles of the present disclosure.

### DETAILED DESCRIPTION OF VARIOUS EMBODIMENTS

Particular embodiments of the present disclosure will be described herein with reference to the accompanying drawings. As shown in the drawings and as described throughout the following description, and as is traditional when referring to relative positioning on an object, the term "proximal" refers to the portion of the apparatus that is closer to the user and the term "distal" refers to the portion of the apparatus that is farther from the user. In the following description, well-known functions or constructions are not described in detail to avoid obscuring the present disclosure in unnecessary detail.

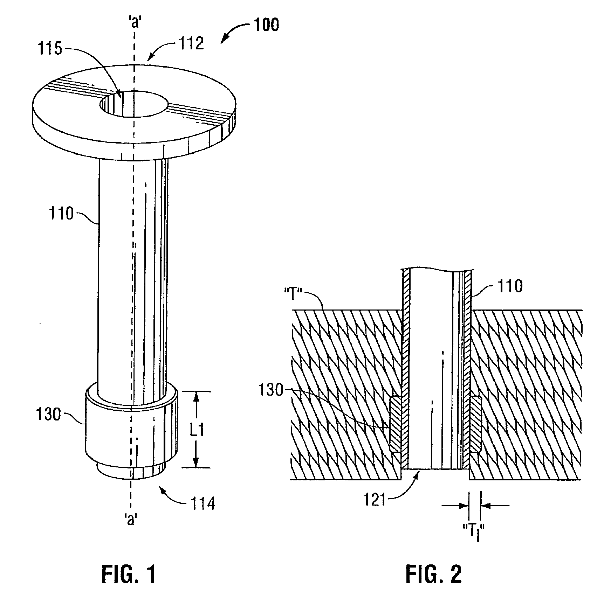

With reference to FIG. 1, a surgical portal device 100 in accordance with embodiments of the present disclosure is shown. The surgical portal device 100 includes a body portion 110 and a securing member 130. The body portion 110 includes a proximal end 112, a distal end 114, and a cylindrical bore or lumen 115 extending therethrough and defining a longitudinal axis "a." The lumen 115 is dimensioned for reception of at least one surgical instrument (not shown), including, but not limited to, clip appliers, graspers, dissectors, retractors, staplers, laser probes, photographic devices, endoscopes and laparoscopes, tubes, and the like.

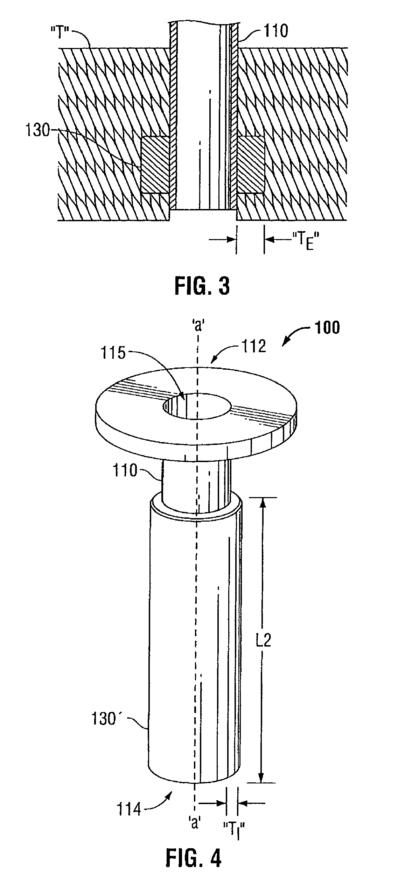

The securing member 130, illustrated in FIGS. 1-3 as being mounted about a circumference of the body portion 110 adjacent its distal end 114, is radially expandable from an initial position (FIGS. 1 and 2) to an expanded position (FIG. 3). The securing member 130 includes a hydrophilic material such that the securing member 130 is configured to radially expand in response to contact with moisture (e.g., tissue; See FIG. 2). As can be appreciated, the radial expansion of the securing member 130 after the securing member 130 contacts moisture in tissue, helps secure the surgical portal device 100 within the patient's tissue. More particularly, during insertion of the surgical portal device 100, the securing member 130 is in its initial, non-expanded position (FIGS. 1 and 2), thus facilitating entry into an incision in a patient. After insertion, when the hydrophilic material contacts the moisture from the patient's tissue, the securing member 130 radially expands (FIG. 3) into adjacent tissue and thus exerting outward pressure thereagainst. This outward pressure helps resist proximal and distal forces exerted on the surgical portal device 100, thus helping to maintain the surgical portal device 100 within tissue.

It is envisioned that the hydrophilic materials of the present disclosure include polymer structures capable of taking in water such that the properties of the polymer system are reproducibly and stably modified. During this process, the materials normally swell isotropically without dissolving or otherwise degrading. Typical conventional materials include hydroxyethyl methacrylate (HEMA) and co-polymers of HEMA or methyl methacrylate with vinyl pyrrolidone (or 1-vinyl-2-pyrrolidinone). Such materials absorb water in large quantities, e.g. between about 0.5 and about 5 times the dry weight of the polymer. The result is that the polymer, initially hard, becomes soft and elastic and exhibits much enhanced water and gas permeabilities.

In various embodiments, the securing member 130 also includes a medicinal agent, a pain reliever, an anti-inflammatory, an antibiotic or any suitable combinations thereof. In such embodiments, the securing member 130 may release these materials into the patient's tissue, thus resulting in less pain, quicker healing, etc. It is also envisioned that the securing member 130 includes hydrogel therein or includes a hydrogel coating, e.g., to help clean and debride necrotic tissue.

The securing member 130 may be configured as an outer shell including the hydrophilic material therein. In such embodiments, the outer shell may be elastomeric to allow for the expansion of the hydrophilic material. Additionally, the outer shell may include pores (not shown) disposed along an outer perimeter thereof, such that the pores facilitate moisture to contact the hydrophilic material.

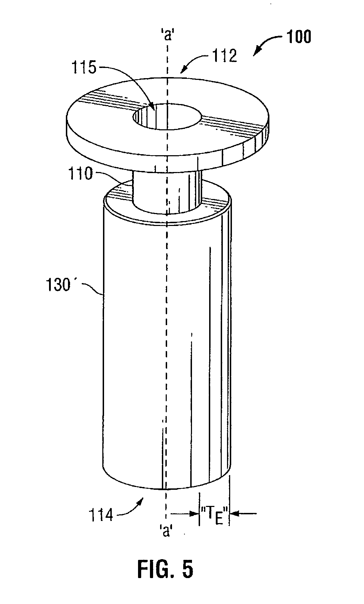

It is envisioned that the securing member 130 has any suitable length. As such, and with reference to FIGS. 1-3, the securing member 130 may be ring-like, such that the longitudinal length "L1" of the securing member 130 is less than its circumference. In the embodiment illustrated in FIGS. 4 and 5, the securing member 130' is sleeve-like, such that the longitudinal length "L2" of the securing member 130' is greater than its circumference. Additionally, the initial thickness "T_{I}" (FIGS. 1, 2 and 4) and the expanded thickness "T_{E}" (FIGS. 3 and 5) of the securing member 130, 130' may each be any suitable thickness. The thicknesses "T_{I}" and "T_{E}" shown in the illustrated embodiments are for illustrative purposes and are not intended to be limiting. For example, the "T_{I}" of the securing member 130 may be close to zero in the embodiments where the securing member 130 includes a hydrogel coating, for example.

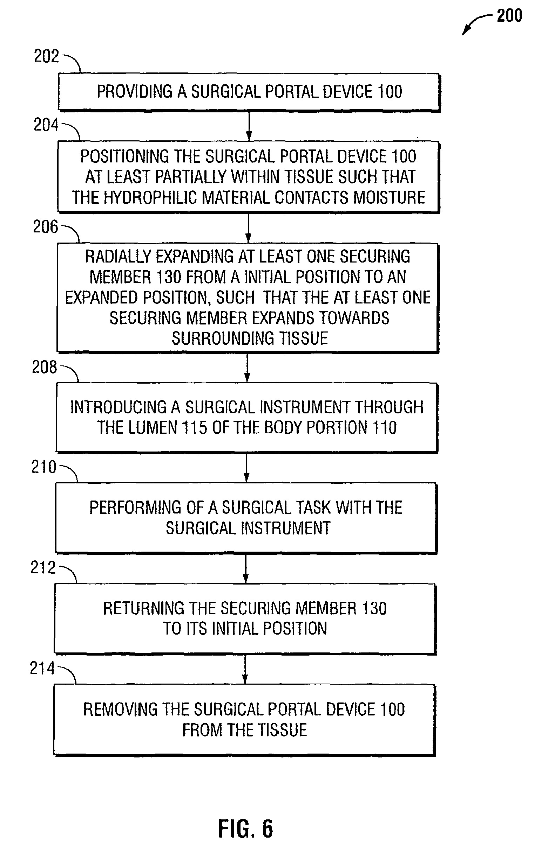

The present disclosure also relates to surgical methods utilizing the surgical portal device 100. FIG. 6 is a flow chart illustrating a method 200 of use of the surgical portal device 100. In accordance with the method 200, the surgical portal device 100 is provided (Step 202). The surgical portal device 100 is positioned at least partially within tissue such that the hydrophilic material contacts moisture (Step 204). At least one securing member 130 is radially expanded from a initial position to an expanded position, such that the at least one securing member expands towards surrounding tissue (Step 206). A surgical instrument is introduced through the lumen 115 of the body portion 110 (Step 208) followed by performance of a surgical task with the surgical instrument (Step 210). The securing member 130 may then be returned to its initial position (Step 212) and the surgical portal device 100 may be removed from the tissue (Step 214). It is envisioned that the surgical portal device 100 may be removed from the tissue in response to the exertion of a proximal force on the body portion 110 by a clinician. **[please confirm accuracy of removal of device 100]**

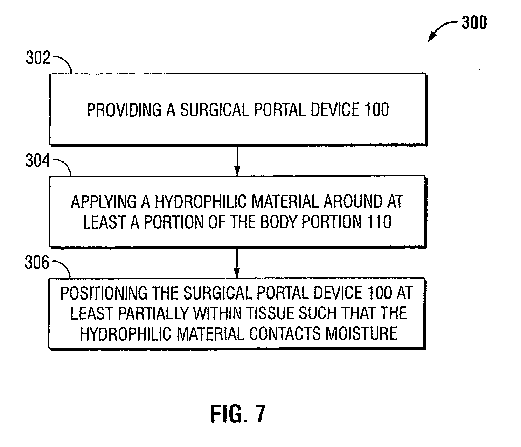

FIG. 7 is a flow chart illustrating a method 300 of use of the surgical portal device 100. In accordance with the method 300, the surgical portal device 100 is provided (Step 302). Hydrophilic material is applied around at least a portion of the body portion 110 (Step 304). The surgical portal device 100 is positioned at least partially within tissue such that the hydrophilic material contacts moisture (Step 306).

It will be understood that various modifications may be made to the embodiments of the presently disclosed portal device. Therefore, the above description should not be construed as limiting, but merely as exemplifications of embodiments. Those skilled in the art will envision other modifications within the scope and spirit of the present disclosure.

## Claims

1. A surgical portal device, comprising:
a body portion defining a longitudinal axis and having a proximal end, a distal end, and a lumen configured to allow a surgical instrument to pass therethrough; and
at least one securing member disposed in mechanical cooperation with the body portion and being radially expandable with respect to the body portion between an initial position and an expanded position, the at least one securing member including a hydrophilic material and being configured to radially expand in response to contact with moisture.

2. The surgical portal device of claim 1, wherein the securing member comprises at least one of a medicinal agent, a pain reliever, an anti-inflammatory, and an antibiotic.

3. The surgical portal device of claim 1 or claim 2, wherein the securing member comprises a hydrogel coating.

4. The surgical portal device of any preceding claim, wherein the at least one securing member extends around an outer perimeter of the body portion.

5. The surgical portal device of any preceding claim, wherein the at least one securing member is sleeve-like.

6. The surgical portal device of any of claims 1 to 4, wherein the at least one securing member is ring-like.

7. A surgical portal device, including:
a body portion defining a longitudinal axis;
a lumen extending through the body; and
at least one securing member capable of being disposed in mechanical cooperation with a body portion, the at least one securing member including a hydrophilic material; for use in a surgical method, wherein the surgical method comprises the steps of:
positioning the surgical portal device at least partially within tissue such that the hydrophilic material contacts moisture;
radially expanding the at least one securing member from an initial position to an expanded position, such that the at least one securing member expands towards surrounding tissue;
introducing a surgical instrument through the lumen of the body portion; and
performing a surgical task with the surgical instrument.

8. The surgical portal device for use according to claim 7, further comprising the step of returning the at least one securing member to its initial position.

9. The surgical portal device for use according to claim 7 or claim 8, further comprising the step of removing the surgical portal device from the tissue.

10. The surgical portal device for use according to any of claims 7 to 9, wherein the radially expanding step occurs in response to the at least one securing member contacting moisture.

11. The surgical portal device for use according to any of claims 7 to 10, wherein the securing member comprises at least one of a medicinal agent, a pain reliever, an anti-inflammatory, and an antibiotic.

12. A method of securing a portion of a surgical portal device adjacent tissue, the method comprising the steps of:
providing a surgical portal device including:
a body defining a longitudinal axis; and
a lumen extending through the body;
applying a hydrophilic material around at least a portion of the body portion; and positioning the surgical portal device at least partially within tissue such that the hydrophilic material contacts moisture.

13. The method of claim 12, wherein the securing member comprises at least one of a medicinal agent, a pain reliever, an anti-inflammatory, and an anti-biotic.
